(19) Europäisches Patentamt
European Patent Office
Office européen des brevets

(11) Veröffentlichungsnummer: **0 003 295**
**B1**

(12) # EUROPÄISCHE PATENTSCHRIFT

(45) Veröffentlichungstag der Patentschrift: **25.11.81**

(21) Anmeldenummer: **79100087.0**

(22) Anmeldetag: **12.01.79**

(51) Int. Cl.³: **C 07 C 59/66,**
C 07 C 69/734,
C 07 C 121/75,
A 01 N 37/38, A 01 N 33/04

(54) **Neue Phenoxy-phenoxy-alkancarbonsäurederivate mit herbizider Wirkung, deren Herstellung, sie enthaltende Mittel und deren Verwendung.**

(30) Priorität: **20.01.78 CH 624/78**

(43) Veröffentlichungstag der Anmeldung:
**08.08.79 Patentblatt 79/16**

(45) Bekanntmachung des Hinweises auf die Patenterteilung:
**25.11.81 Patentblatt 81/47**

(84) Benannte Vertragsstaaten:
**BE CH DE FR GB IT NL**

(56) Entgegenhaltungen:
**EP 79 10 0087**
**FR - A - 2 325 638**

(73) Patentinhaber: **CIBA-GEIGY AG**
**Patentabteilung Postfach**
**CH-4002 Basel (CH)**

(72) Erfinder: **Rohr, Otto Dr.**
**Kilbertweg 19**
**CH-4106 Therwil (CH)**
Erfinder: **Pissiotas, George, Dr.**
**Breslauerstrasse 8**
**D-7850 Lörrach (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 003 295 B1

Courier Press, Leamington Spa, England.

Neue Phenoxy-phenoxy-alkancarbonsäurederivate mit herbizider Wirkung, deren Herstellung, sie enthaltende Mittel und deren Verwendung

Die vorliegende Erfindung betrifft neue Phenoxy-phenoxy-alkancarbonsäurederivate, welche herbizide und das Pflanzenwachstum regulierende Wirkung haben, Verfahren zu deren Herstellung, Mittel welche diese Derivate als Wirkstoffe enthalten sowie die Verwendung dieser Phenoxy-phenoxy-alkancarbonsäurederivate oder sie enthaltender Mittel als Herbizide oder zur Regulierung des Pflanzenwachstums.

Die Phenoxy-phenoxy-alkancarbonsäurederivate sind neue Verbindungen und entsprechen der Formel I:

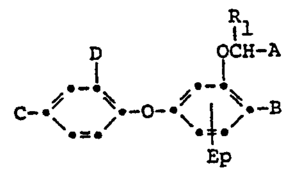

(I)

In dieser Formel bedeuten

$R_1$ Wasserstoff, $C_1$—$C_4$ Alkyl, $C_2$—$C_3$ Alkoxyalkyl

A die Cyanogruppe oder einen Rest —COR,

R ein Rest $—OR_3$, $SR_4$, $—NR_5R_6$ oder $—N{=}R_2$

$R_3$ Wasserstoff oder das Kation einer Base $\frac{1}{m} M^{m\oplus}$

M ein Alkali-, Erdalkali-Kation oder ein Fe, Cu-, Zn-, Mn, Ni-Kation oder einen Ammonio-Rest

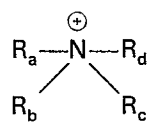

m als ganze Zahl 1, 2 oder 3 die Wertigkeit des Kations berücksichtigt, während $R_a$, $R_b$, $R_c$ und $R_d$ unabhängig voneinander Wasserstoff, Benzyl oder einen gegebenenfalls durch —OH, $—NH_2$ oder $C_1$—$C_4$ Alkoxy substituierten $C_1$—$C_4$ Alkylrest bedeuten,

weiter bedeuten

$R_3$ einen $C_1$—$C_{12}$ Alkyl-Rest, der unsubstituiert oder gegebenenfalls substituiert ist durch Halogen, Nitro, Cyano, $C_1$—$C_8$ Alkoxy, $C_1$—$C_8$ Alkylthio, $C_2$—$C_8$ Alkanoyl, Bis ($C_1$—$C_4$ alkyl) amino, Tris ($C_1$—$C_4$ alkyl)ammonio, $C_3$—$C_8$ Cycloalkyl, $C_3$—$C_8$ Cycloalkenyl, gegebenenfalls auch durch einen unsubstituierten oder seinerseits durch Halogen, $C_1$—$C_4$-Alkyl, $C_1$—$C_4$-Alkoxy ein- oder mehrfach substituierten Phenyl-, Phenoxy-oder 5—6 gliedrigen heterocyclischen Rest mit 1 bis 3 Heteroatomen;

— einen unsubst. oder subst. $C_3$—$C_{18}$ Alkenylrest;

— einen $C_3$—$C_8$ Alkinyl-Rest;

— einen gegebenenfalls durch Halogen oder $C_1$—$C_4$-Alkyl substituierten $C_3$—$C_{12}$ Cycloalkyl-Rest;

— einen Phenylrest, der unsubstituiert oder durch Halogen, $C_1$—$C_4$-Alkyl, $C_1$—$C_4$-Alkoxy, $C_1$—$C_4$-Alkylthio, $NO_2$, $CF_3$, ein- oder mehrfach substituiert ist;

— einen 5- bis 6-gliedrigen heterocyclischen Ring mit 1 bis 3 Heteroatomen,

$R_4$ dasselbe wie $R_3$,

$R_5$ und $R_6$ unabhängig voneinander je Wasserstoff oder einen *Alkylrest* der gegebenenfalls durch Hydroxyl oder Alkoxy substituiert sein kann, *Alkoxy, Alkenyl, Alkinyl, Phenyl* oder *Benzyl* wobei die Phenylringe ein- oder mehrfach durch Halogen, Alkyl, Alkoxy, Alkylthio, Cyano oder Nitro substituiert sein können oder

$R_5$ und $R_6$ zusammen mit dem Stickstoffatom, an das sie gebunden sind, einen 5—6 gliedrigen Heterocyclus, der auch noch ein zweites Heteroatom, Stickstoff, Sauerstoff oder Schwefel enthalten kann,

$R_2$ einen durch eine Doppelbindung an das Stickstoffatom gebunden, vorzugsweise verzweigten Alkylenrest,

B ein Wasserstoff oder Halogenatom oder einen Cyano- oder Nitrorest, und

C und D je Wasserstoff, Halogen oder einen Cyano-, Nitro- oder Trifluoromethylrest,

E ein Halogenatom oder ein $C_1$—$C_4$ Alkylrest,

P eine Zahl 1 oder 2 bedeuten.

In dieser Formel sind die Alkylreste sowohl verzweigt wie unverzweigt und enthalten die gegebene Anzahl Kohlenstoffatome. Die Reste $R_5$ und $R_6$ sind bevorzugt Wasserstoff und $C_1$—$C_4$ Alkylreste. Einzelne unter ihnen können jedoch auch substituierte Alkylreste bedeuten oder eines davon

einen gegebenenfalls substituierter Phenylrest, oder Benzyl sein oder sie können zusammen mit dem Stickstoffatom an das sie gebunden sind auch einen Heterocyclus bilden, der vorzugsweise 5—6 Ringglieder enthält.

Diese Erfindung erschliesst eine neue Gruppe von Phenoxy-phenoxy- resp. Phenoxy-phenylthioalkancarbonsäure-Derivate, die bei geringen Aufwandmengen bereits starke herbizide Wirkung zeigen und/oder das Pflanzenwachstum in anderer, für die Landwirtschaft nützlicher Weise zu regulieren vermögen.

Die neuen Wirkstoffe der Formel I können als Herbizide im Vor- und hauptsächlich im Nachauflaufverfahren eingesetzt werden. Sie wirken gut gegen breitblättrige Pflanzen (Dikotyledonen).

Die erfindungsgemässen Derivate besitzen auch günstige wachstumregulierende Wirkung (Wuchshemmung). Sie hemmen das Wachstum von vor allem dicotylen Pflanzen und können in nutzbringender Weise z.B. wie folgt eingesetzt werden:

— Reduktion des vegetativen Wachstums bei Soja und ähnlichen Leguminosen, was zu einer Ertragssteigerung dieser Kultur führt;

— Hemmung des unerwünschten Wachstums von Geiztrieben bei Tabak, dessen Haupttrieb man geschnitten hat, was der Ausbildung grösserer und schönerer Blätter zugute kommt;

— Hemmung des Wachstums von Gras und dikotyledonen Pflanzen, wie Obstbäume, Zierbäume, Gebüsche und Hecken, zwecks Einsparung an Schnittarbeit.

Die Verbindungen vorliegender Erfindung sind wenig giftig für Warmblüter und deren Applikation dürfte keine Probleme verursachen. Die vorgeschlagenen Aufwandmengen liegen zwischen 0.1 und 5 kg pro Hektar.

Phenoxy-phenoxy-alkancarbonsäure-derivate mit herbizider Wirkung und ähnlicher chemischer Struktur sind bereits aus einer Reihe neuerer Veröffentlichungen bekannt geworden. Wir möchten auf die Deutschen Offenlegungsschriften No. 2 311 638, 2 609 461, 2 623 558, 2 639 796, 2 643 438 (FR - A - 2 325 638), 2 652 384, 2 730 591, sowie den C. A. *86* (1977) 134 874x verweisen. Die vorliegenden Verbindungen sind verschieden von denen, welche in diesen Veröffentlichungen genannt sind, und zeigen trotz ihrer ausgesprochenen Wirkung auf dicotyle Unkräuter einen herausragende Selektivität auf breitblättrige Kulturpflanzen wie Zuckerrüben und Soja.

Die Herstellung der neuen Verbindungen der Formel I erfolgt nach den zur Synthese von Phenoxy-phenoxy-alkan-carbonsäuren und deren Derivaten an sich bekannten Methoden.

Nach einem ersten dieser Verfahren setzt man erfindungsgemäss ein entsprechend substituiertes Halogen-phenol der Formel II

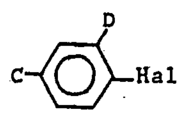

(II)

worin Hal ein Halogenatom, vorzugsweise Chlor oder Brom bedeutet und C, und D die unter Formel I gegebene Bedeutung haben, mit einem entsprechend substituierten Hydroxy-phenoxy-alkanosäurederivat der Formel III

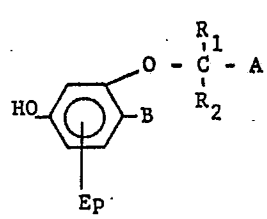

(III)

worin B, E, p, $R_1$, $R_2$ und A die unter Formel I gegebene Bedeutung haben, in Gegenwart einer Base um.

Nach einem zweiten Verfahren geht man erfindungsgemäss von einem entsprechend substituierten m-Hydroxydiphenyläther der Formel IV

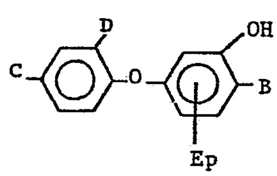

(IV)

worin B, C, D, E, und p die unter Formel I gegebene Bedeutung haben aus und setzt diesen mit einem $\alpha$-Halogenalkansäurederivat der Formel V

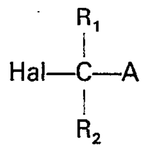

worin Hal ein Halogenatom, vorzugsweise Chlor oder Brom bedeutet und $R_1$, $R_2$ und A die unter Formel I gegebene Bedeutung haben, in Gegenwart einer Base um.

Falls man bei diesen Verfahren als Ausgangsstoffe (A=COOH) der Formeln III bezw. V eine Carbonsäure verwendet, so kann man nachträglich z.B. durch Veresterung etc. diese Gruppe in ein anderes definitions-gemässes Derivat der Formel I überführen. Umgekehrt kann man bei Verwendung eines Esters der Formeln III bezw. V abschliessend durch Verseifung die Estergruppe in die freie Carbonsäure oder ein Salz derselben überführen.

Die genannten Umsetzungen können in An- oder Abwesenheit von gegenüber den Reaktionsteilnehmern inerten Lösungs- oder Verdünnungsmitteln durchgeführt werden. Bevorzugt sind polare organische Lösungsmittel wie Methyläthylketon, Dimethylformamid, Dimethylsulfoxid etc. Die Reaktions- temperaturen liegen zwischen 0 und 200° und die Reaktions- dauer beträgt je nach Ausgangsstoff, gewählter Umsetzungstemperatur und Lösungsmittel zwischen 1 Stunde und mehreren Tagen. Man arbeitet in der Regel bei Normaldruck. Als Basen (Kondensationsmittel) für die Umsetzung kommen die üblichen, wie z.B. KOH, $NaOCH_3$, $NaHCO_3$, $K_2CO_3$, Kalium-tert. butylat etc. aber auch organische Basen wie Triäthylamin etc. in Betracht.

Die Ausgangsstoffe der Formel II bis V sind teilweise bekannt. Noch nicht beschriebene Ausgangsstoffe dieser Formeln lassen sich nach üblichen Verfahren und Techniken leicht herstellen. Phenoxyphenole der Formel IV können beispielsweise gemäss den im J.A.C.S. *61*, 2702 (1939) oder in Chem. Abstracts *54*, $922^h$ (1960) beschriebenen Methoden hergestellt werden.

Die nachfolgenden Beispiele veranschaulichen die erfindungsgemässen Verfahren. Weitere in entsprechender Weise hergestellte Wirkstoffe sind in der anschliessenden Tabelle aufgeführt. Temperaturangaben beziehen sich auf Celsius-Grade, Prozente auf das Gewicht.

## Beispiel 1

α-[3-(2′-Chlor-4′-trifluormethylphenoxy)-4-chlorphenoxy]-propionsäure-methylester

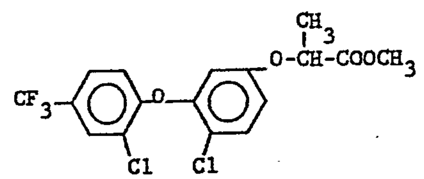

a) 11,5 g 2-Chlor-4-trifluormethyl-2′-chlor-5′-hydroxydiphenyläther werden in 50 ml 2-Butanon gelöst und nach Zugabe von 30 ml 2-Brompropionsäure-methylester, 12 g Kaliumcarbonat und einer Spatelspitze Kaliumjodid über Nacht bei 80°C gerührt. Das Reaktionsprodukt wird mit 100 ml Aceton verdünnt und abfiltriert. Nach Abdestillieren des Lösungsmittels wird der Rückstand im Hochvakuum destilliert. Man erhält 12,8 g des oben etwähnten Produktes welches einen Siedepunkt von 147°C bei 0.04 Torr hat.

Das Ausgangsprodukt, 2-Chlor-4-trifluormethyl-2-′-chlor-5′-hydroxy-diphenyläther wurde wie folgt hergestellt:

b) 20 g (0,33 Mol) Kaliumhydroxyd pulverisiert werden in 200 ml Dimethylsulfoxid vorgelegt. Dazu gibt man portionenweise 48 g (0,3 Mol) 2-Chlor-3-methoxyphenol. Nachdem alles in Lösung gegangen ist tropft man ziemlich rasch 70 g (0,33 Mol) 3,4-Dichlorbenzotrifluorid zu und rührt während ca. 12 Std. bei 150°C (Badtemperatur) Das Reaktionsprodukt wird in Eis/Wasser gegeossen und mit Toluol/Essigester 1:1 extrahiert. Nach Abdestillieren des Lösungsmittels erhält man 91 g 2-Chlor-4-trifluormethyl-2′-chlor-5′-methoxy-diphenyläther als Oel, das im Hochvakuum destilliert wird. Siedepunkt 119°—123°C bei 0,25 Torr. Ausbeute 59,5 g (59% der Theorie).

c) 32 g 2-Chlor-4-trifluormethyl-2′-chlor-5′-methoxydiphenyläther werden in 700 ml Eisessig gelöst und mit 170 ml 48%iger Bromwasserstoffsäure versetzt. Die Mischung wird während 60 Std. unter Rühren bei 120°C Badtemperatur am Rückfluss gekocht und anschliessend wird der Eisessig abdestilliert. Das zurückbleibende Oel wird in Toluol aufgenommen und mit einer Lösung von Natriumbikarbonat/Natriumthiosulfat gewaschen. Nach Wegdestillieren des Lösungsmittels verbleiben 28 g 2-Chlor-4-trifluormethyl-2′-chlor-5′-hydroxy-diphenyläther als viskoses Oel, das im Hochvakuum destilliert wird. Ausbeute 19,5 g, Siedepunkt 132°C bei 0,25 Torr.

Beispiel 2

α-[3-(4'-Trifluormethylphenoxy)-4,6-dichlorphenoxy]propionsäure-methylester

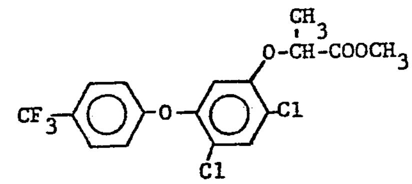

34 g (0,1 Mol) 3-(4'-Trifluormethylphenoxy)-α-(phenoxy)-propionsäure-methylester (analog Beispiel 1 hergestellt) werden in 100 ml Eisessig gelöst und bei 40°C im Ueberschuss Chlor eingeleitet. Die Reaktion wird gaschromatographisch verfolgt. Nach beendeter Reaktion wird noch 1/2 Std. ausgerührt und überschüssiges Chlor durch Einleiten von Stickstoff entfernt. Das Lösungsmittel wird abdestilliert und das so erhaltene Oel im Hochvakuum destilliert. Man erhält so 34,5 g des obigen Produktes als Oel, welches im Hochvakuum destilliert wird, Siedepunkt 163—165°C bei 0,07 Torr.

Beispiel 3

α-[3-(4'-Trifluormethylphenoxy)-5-methylphenoxy]propionsäure-methylester

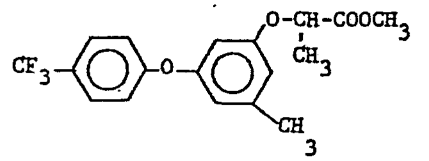

a) 13,0 g (0,0485 Mol) 5-(4-Trifluormethylphenoxy)-m-kresol) werden mit 7,6 g (0,055 Mol) Kaliumcarbonat und 50 ml Methyläthylketon vorgelegt und für 2 Std. am Rückfluss gerührt. Dann gibt man bei 50°C 8,4 g (0,05 Mol) 2-Brompropionsäuremethylester zu und lässt 15 Std. bei 50° ausrühren. Nach dem Abkühlen wird abfiltriert und das Filtrat am Vakuum eingedampft. Den Rückstand filtriert man mit Methylenchlorid über eine kleine Säule ab. Dabei erhält man 16,1 g (93,6% d.Th) des obigen Produktes als hellgelbes, klares Oel mit Brechungsindex $n_D^{20}$ 1,5104. Das Ausgangsmaterial, 5-(4-Trifluormethyl-phenoxy-m-kresol wird wie folgt hergestellt.

b) 35,5 g (0,25 Mol) Orcin·$H_2O$ (3,5-Dihydroxytoluol) werden in 200 ml Dimethylsulfoxyd gelöst und mit 31,3 g (0,5 Mol) Kaliumhydroxyd (90%), das fein pulverisiert ist, versetzt. Sobald alles in Lösung gegangen ist, gibt man 45,1 g (0,25 Mol) 4-Chlorbenzoltrifluorid zu und lässt das Reaktionsgemisch für 20 Std. bei 150° ausrühren. Nach dem Abkühlen fügt man 200 ml HCl 10% zu und extrahiert mit Methylenchlorid. Die organische Phase wird getrocknet und eingedampft. Der Rückstand wird über einer kleinen Kieselgelsäule mit Methylenchlorid aufgetrennt. Als Hauptfraktion erhält man 29,8 g (44,4% d.Th.) 5-(4-Trifluormethylphenoxy)-m-kresol als hellgelbes, klares Oel mit dem Brechungsindex $n_D^{20}$ 1,5322.

Beispiel 4

α-[3-(4'-Trifluormethylphenoxy)-5-methyl-6-chlorphenoxy]propionsäure-methylester

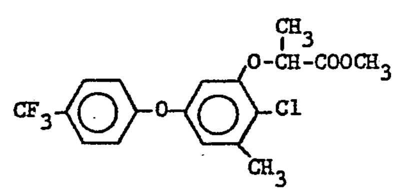

a) 1,7 g (0,0056 Mol) 5-(4'-Trifluormethylphenoxy)-2-chlor-m-kresol werden mit 0,23 g (0,0062 Mol) Kaliumcarbonat in 20 ml Aethylmethylketon während 2 Std. am Rückfluss gerührt. Dann gibt man bei 50°C 1,0 g (0,0062 Mol) 2-Brompropionsäuremethylester zu und lässt 15 Std. bei 60°C ausrühren. Anschliessend filtriert man ab und dampft das Filtrat ein. Den Rückstand filtriert man mit Methylenchlorid über eine kleine Säule und erhält beim Eindampfen der Hauptfraktion 1,6 g (76,2% der Theorie) des obigen Produktes als helles Oel mit dem Brechungsindex $n_D^{20}$ 1,5119.

Das als Ausgangsmaterial verwendete 5-(4'-Trifluormethylphenoxy)-2-chlor-m-kresol wird wie folgt hergestellt:

b) 13,4 g (0,05 Mol) 5-(4-Trifluormethylphenoxy)-m-kresol (Herstellung siehe Beispiel 3b) werden, in 50 ml Eisessig gelöst, vorgelegt. Zu dieser Lösung tropft man bei 15° 3,8 g Chlor (0,055 Mol) in 25 ml Eisessig. Dabei hält man die Temperatur bei 15°. Anschliessend lässt man die Innentemperatur auf Raumtemperatur steigen. Nach 15 Min. giesst man das Reaktionsgemisch auf Wasser, extrahiert mit Methylenchlorid, trocknet die organische Phase mit Natriumsulfat und dampft sie am Vakuum ein. Der Rückstand wird mit Hexan über eine kleine Kieselgelsäule chromatographiert. Die Hauptracktion ergibt ein klares, helles Oel, das beim Zerreiben mit Petroläther kristallisiert. So erhält man 1,7 g (11,3% d. Th.) 5-(4-Trifluormethyl-phenoxy)-2-chlor-m-kresol als weisses Produkt mit dem Schmelzpunkt 106—10°C.

Beispiel 5

α-[3-(4′-Trifluormethylphenoxy)-5-methyl]-propionitril

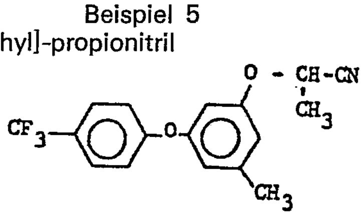

13,0 g (0,0485 Mol) 5-(4′-Trifluormethyl-phenoxy)-m-kresol (Herstellung siehe Beispiel 3b) werden mit 7,6 g (0,055 Mol) Kaliumcarbonat und 50 ml Methyläthylketon vorgelegt und für 2 Std. am Rückfluss gerührt. Dann gibt man bei 40° 6,7 g (0,05 Mol) 2-Brompropionitril zu und lässt 15 Std. bei 60° ausrühren. Nach dem Abkühlen wird abfiltriert und das Filtrat am Vakuum eingedampft. Den Rückstand filtriert man mit Methylenchlorid über eine kleine Kieselgelsäule ab. Dabei erhält man 14,4 g (90,0% der Theorie) des obigen Produktes als braunes, klares Oel mit dem Brechungsindex $n_D^{20}$ 1,5174.

Beispiel 6

α-[3-(4′-Trifluormethylphenyl)-5-methylphenoxy]propionsäure

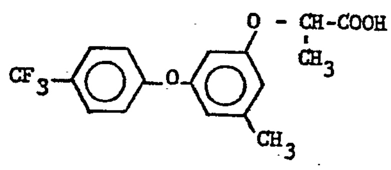

39,0 g (0,11 Mol) α-[3-(4′-Trifluormethylphenoxy)-3-methylphenoxy]-propionsäure-methylester (Herstellung siehe Beispiel 3a) werden in 100 ml Methanol gelöst und mit einer Lösung von 10,1 g Natriumhydroxyd 100% in 10 ml Wasser versetzt. Dabei steigt die Temperatur an. Man lässt noch 1 Std. bei 50° nachrühren, versetzt das Reaktionsgemisch mit 100 ml Eis/Wasser und säuert mit konc. Salzsäure an. Das ausgefallene Produkt wird in 200 ml Methylenchlorid aufgenommen und getrocknet.

Eingedampft ergibt es 23,8 g (63,4%) der Theorie des obigen Produktes als hellgelbes kristallines Pulver mit dem Schmelzpunkt 88°—90°.

Beispiel 7

α-[3-(4′-Trifluormethylphenoxy)-5-methylphenoxy]propionsäure-allylamid

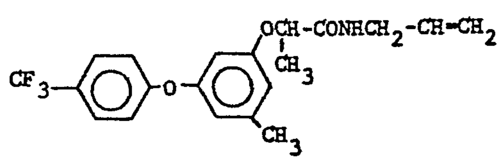

a) 7 g (0,019 Mol) α-[3-(4′-Trifluormethylphenoxy)-5-methylphenoxy-propionsäurechlorid werden in 20 ml Methylenchlorid gelöst und bei 10° mit 2,8 g (0,042 Mol) Allylamin versetzt.

Nach 1 Stunde gibt man 100 ml Wasser zu, trennt die organische Phase ab und trocknet diese mit Natriumsulfat. Beim Eindampfen gewinnt man ein hellgelbes, klares Oel, das beim Zerreiben mit Petroläther kristallisiert. So erhält man das obige Produkt als weisse Kristalle in einer Ausbeute von 5,0 g (69,4% der Theorie). Schmelzpunkt 94—96°. Das als Ausgangsmaterial verwendete Säurechlorid wurde wie folgt hergestellt:

b) 13,8 g (0,04 Mol) α-(3-(4′-Trifluormethylphenoxy)-5-methylphenoxy)-propionsäure (Herstellung siehe Beispiel 6) werden bei Raumtemperatur mit 20 ml Thionylchlorid versetzt und auf 50° erwärmt. Nach 2 stündigem stehelassen bei dieser Temperatur dampft man das Reaktionsgemisch ein, versetzt den Rückstand mit 50 ml Toluol und dampft bei gutem Vakuum bis zur Trockene ein. Dabei erhält man als dunkelbraunes, klares Oel 14,3 g (100% d. Th.) α-(3-(4′-Trifluormethylphenoxy)-5-methylphenoxy)-propionsäurechlorid.

Beispiel 8

α-[3-(2'-Chlor-4'-trifluormethylphenoxy)-6-chlorphenoxy]propionsäure-methylester

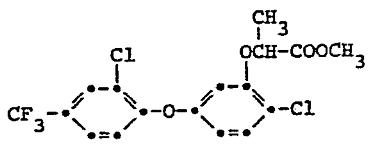

35,9 g 3-(2'-Chlor-4'-trifluormethyl-phenoxy)-α-phenoxypropionsäure-methylester werden in 100 ml Eisessig gelöst. In diese Lösung leitet man unter gutem Rühren die theoretisch nötige Menge Chlor ein, wobei die Temperatur auf 40°C gehalten wird. Es wird eine halbe Stunde ausgerührt und das Lösungsmittel abdestilliert. Der ölige Rückstand wird mit Benzin versetzt und die ausgefallenen Kristalle abfiltriert. Das so erhaltene Titelprodukt hat einen Schmelzpunkt von 79°—80°.

Beispiel 9

α-[3-(2'-Chlor-4'-trifluoromethyl)-6-chlor-4-jodphenoxy)propionsäure-methylester

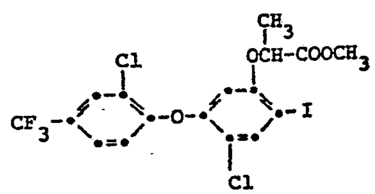

41 g 3-(2'-Chlor-4'-trifluormethylphenoxy)-α-6-chlorphenoxypropionsäure-methylester werden in 150 ml Eisessig gelöst. Dazu tropft man eine Lösung von 20,5 Chlorjod in 30 ml Eisessig, lässt 15 Minuten ausreagieren (schwach exotherme Reaktion) und erhitzt nach Zugabe von 200 ml Wasser während 3 Stunden auf 90°. Darauf gibt man eine 5%ige Natriumbisulfitlösung bis zur Entfärbung zu und extrahiert das ausgefallene Oel mit Toluol/Essigester 1:1. Nach Abdestillieren des Lösungsmittels erhält man 47 g Oel, das nach Zugabe von Benzin kristallisiert. Nach Abfiltrieren des Kristallbreis erhält man 32 g Titelprodukt als weisse kristalline Masse vom Schmelzpunkt 102°—105°.

Beispiel 10

α-[3-(2'-Chlor-4'-trifluormethylphenoxy)-6-chlor-4-cyanophenoxy]-propionsäure-methylester

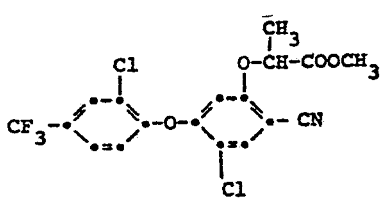

27 g 3-(2'-Chlor-4'-trifluormethylphenoxy)-α-6-chlor-4-Jodphenoxy-propionsäure-methylester werden in 50 ml N-Methyl-2-pyrrolidon gelöst. Dazu gibt man unter langsamem Rühren 6 g Kupfer-I-cyanid. Das Reaktionsgemisch wird unter schwachem Stickstoffstrom auf 140° Badtemperatur erwärmt und während 3 Stunden bei dieser Temperatur gerührt. Nach dem Erkalten wird das ausgeschiedene Kupfer-I-jodid abfiltriert und das Filtrat langsam in eine Mischung von 25 ml 25%igem Ammoniak und 75 g Eis eingetragen. Die ausgeschiedenen Kristalle werden abfiltriert und getrocknet. Man erhält so 21 g kristallines Titelprodukt vom Schmelzpunkt 121°—124°.

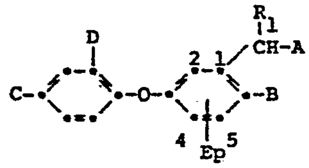

| No. | C | D | B | Ep | $R_1$ | A | physikalische Konstante |
|---|---|---|---|---|---|---|---|
| 1 | $CF_3$ | – | – | 5 $CH_3$ | $CH_3$ | $CONHCH_2CH{=}CH_2$ | Smp. 94–96° |
| 2 | $CF_3$ | – | Cl | 4 Cl | $CH_3$ | $COOCH_3$ | Kp. 163–5°/0.07 Torr |
| 3 | $CF_3$ | – | – | 5 $CH_3$ | $CH_3$ | $COOCH_3$ | $n_D^{20}$ 1.5104 |
| 4 | $CF_3$ | – | Cl | 5 $CH_3$ | $CH_3$ | $COOCH_3$ | $n_D^{20}$ 1.5119 |
| 5 | $CF_3$ | – | – | 5 $CH_3$ | $CH_3$ | CN | $n_D^{20}$ 1.5174 |
| 6 | $CF_3$ | – | – | 5 $CH_3$ | $CH_3$ | COOH | Smp. 88–90° |
| 7 | $CF_3$ | Cl | – | 4 Cl | $CH_3$ | $COOCH_3$ | Kp. 147/0.04 Torr |
| 8 | $CF_3$ | Cl | – | 4 Cl | $CH_3$ | COOH | Smp. 129–132° |
| 9 | $CF_3$ | Cl | – | 5 Cl | $CH_3$ | $COOCH_3$ | Kp. 153-5/0.02 Torr |
| 10 | $CF_3$ | Cl | Cl | 4 Cl | $CH_3$ | $COOCH_3$ | Smp. 79–80° |
| 11 | $CF_3$ | Cl | CN | 4 Cl | $CH_3$ | $COOCH_3$ | Smp. 121°–124° |
| 12 | $CF_3$ | Cl | $NO_2$ | 4 Cl | $CH_3$ | COOH | |
| 13 | $CF_3$ | Cl | $NO_2$ | 4 Cl | $CH_3$ | $COOCH_3$ | Smp. 106–10° |
| 14 | $CF_3$ | Cl | $NO_2$ | 4 Cl | $CH_3$ | COS 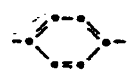 Cl | |
| 15 | $CF_3$ | Cl | $NO_2$ | 4 Br | $CH_3$ | $COOCH_3$ | |
| 16 | $CF_3$ | Cl | $NO_2$ | 4 Br | $CH_3$ | $COSCH_2{-}CH{=}CH_2$ | |
| 17 | $CF_3$ | Cl | $NO_2$ | 4 Br | $CH_3$ | $CONHC_2H_5$ | |
| 18 | $CF_3$ | Cl | CN | 4 Cl | $CH_3$ | CN | Oel |
| 19 | $CF_3$ | Cl | CN | 4 Cl | $CH_3$ | COOH | |
| 20 | $CF_3$ | Cl | CN | 4 Cl | $CH_3$ | $COSC_2H_5$ | |
| 21 | $CF_3$ | Cl | – | 5 $CH_3$ | $CH_3$ | $COOCH_3$ | |
| 22 | $CF_3$ | Cl | – | 5 $CH_3$ | $CH_3$ | COOH | |
| 23 | $CF_3$ | Cl | – | 5 $CH_3$ | $CH_3$ | $COSCH_2{-}CH{=}CH_2$ | |
| 24 | $CF_3$ | Cl | Br | 4 Br | $CH_3$ | $COOCH_3$ | |
| 25 | $CF_3$ | Cl | Br | 4 Br | $CH_3$ | $COOCH_2C{\equiv}CH$ | |
| 26 | $CF_3$ | Cl | Br | 4 Br | $CH_3$ | $COOCH_2$ 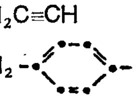 $OCH_3$ | |

| No. | C | D | B | Ep | $R_1$ | A | physikalische Konstante |
|---|---|---|---|---|---|---|---|
| 27 | $CF_3$ | Cl | Cl | 4 Cl | $CH_3$ | $COOC_2H_5$ | Smp. 49°–51° |
| 28 | $CF_3$ | Cl | Cl | 4 Cl | $CH_3$ | $COOCH_2CH{=}CH_2$ | Smp. 47°–50° |
| 29 | $CF_3$ | Cl | Cl | 4 Cl | $CH_3$ | $COSCH_2CH{=}CH_2$ | Oel |
| 30 | $CF_3$ | Cl | Cl | 4 Cl | $CH_3$ | COO iso $C_3H_7$ | |
| 31 | $CF_3$ | Cl | Cl | 4 Cl | $CH_3$ | $CON(CH_3)OCH_3$ | Oel |
| 32 | $CF_3$ | Cl | Cl | 4 Cl | $CH_3$ | COO 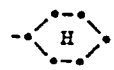 | Smp. 53–55° |
| 33 | $CF_3$ | Cl | Cl | 4 Cl | $CH_3$ | COS 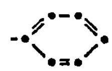 | Smp. 86–89° |
| 34 | $CF_3$ | Cl | Cl | 4 Cl | $CH_3$ | COO 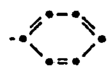 | Smp. 118–119° |
| 35 | $CF_3$ | Cl | Cl | 4 Cl | $CH_3$ | COOH | Smp. 147–149° |
| 36 | $CF_3$ | Cl | Cl | 4 Cl | $CH_3$ | COOsec.$C_4H_9$ | Smp. 49°–52° |
| 37 | $CF_3$ | Cl | Cl | 4 Cl | $CH_3$ | COOiso$C_4H_9$ | Smp. 58°–59° |
| 38 | $CF_3$ | Cl | Cl | 4 Cl | $CH_3$ | $COOCH_2CH(CH_3)C_2H_5$ | Oel |
| 39 | $CF_3$ | Cl | Cl | 4 Cl | $CH_3$ | $COOCH_2C{\equiv}CH$ | Smp. 47–49° |
| 40 | $CF_3$ | Cl | Cl | 4 Cl | $CH_3$ | $COOC_2H_4Cl$ | Oel |
| 41 | $CF_3$ | Cl | Cl | 4 Cl | $CH_3$ | $COON{=}(CH_3)_2$ | Oel |
| 42 | $CF_3$ | Cl | Cl | 4 Cl | $CH_3$ | $COOC_2H_4CN$ | Smp. 75°–79° |
| 43 | $CF_3$ | Cl | Cl | 4 Cl | $CH_3$ | $COOCH(CH_3)CH_2OCH_3$ | Oel |
| 44 | $CF_3$ | Cl | Cl | 4 Cl | $CH_3$ | $COOC_2H_4OCH_3$ | Oel |
| 45 | $CF_3$ | Cl | Cl | 4 Cl | $CH_3$ | $COOC_2H_4N$ 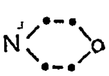 | Oel |
| 46 | $CF_3$ | Cl | Cl | 4 Cl | $CH_3$ | $CONH_2$ | Smp. 94°–96° |
| 47 | $CF_3$ | Cl | Cl | 4 Cl | $CH_3$ | CON 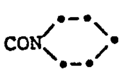 | Smp. 115°–117° |
| 48 | $CF_3$ | Cl | Cl | 4 Cl | $CH_3$ | $CONHC_2H_4OCH_3$ | Smp. 89–91° |

| No. | C | D | B | Ep | $R_1$ | A | physikalische Konstante |
|---|---|---|---|---|---|---|---|
| 49 | $CF_3$ | Cl | Cl | 4 Cl | $CH_3$ | $COOCH_2CF_3$ | Smp. 48°–51° |
| 50 | $CF_3$ | Cl | Cl | 4 Cl | $CH_3$ | CONH - 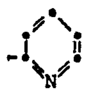 | Smp. 124°–125° |
| 51 | $CF_3$ | Cl | Cl | 4 Cl | $CH_3$ | 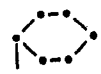 C≡CH | Oel |
| 52 | $CF_3$ | Cl | $NO_2$ | 4 Cl | $CH_3$ | $COSCH_3$ | Oel |
| 53 | $CF_3$ | Cl | – | 4 $NO_2$ | $CH_3$ | $COOCH_3$ | Kp. 190°/0.04 Torr |
| 54 | $CF_3$ | Cl | Br | 4 Cl | $CH_3$ | $COOCH_3$ | Smp. 88–91° |
| 55 | $CF_3$ | Cl | I | 4 Cl | $CH_3$ | $COOCH_3$ | Smp. 102–105° |
| 56 | $CF_3$ | Cl | – 2 | Cl 4 Cl | $CH_3$ | $COOCH_3$ | Kp. 162°/0.05 Torr |
| 57 | $CF_3$ | CN | – | 4 Cl | $CH_3$ | $COOCH_3$ | Smp. 104–108° |
| 58 | $CF_3$ | CN | Cl | 4 Cl | $CH_3$ | $COOCH_3$ | Smp. 105–107° |
| 59 | $CF_3$ | $NO_2$ | – | 4 Cl | $CH_3$ | $COOCH_3$ | Kp. 183–5°/0.05 Torr |
| 60 | $CF_3$ | $NO_2$ | Cl | 4 Cl | $CH_3$ | CN | Oel |
| 61 | $CF_3$ | $NO_2$ | Cl | 4 Cl | $CH_3$ | COOH | |
| 62 | $CF_3$ | $NO_2$ | Cl | 4 Cl | $CH_3$ | COS - 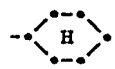 | |
| 63 | $CF_3$ | $NO_2$ | $NO_2$ | 4 Cl | $CH_3$ | $COOCH_3$ | Kp >200°/0.01 Torr |
| 64 | $CF_3$ | $NO_2$ | $NO_2$ | 4 Cl | $CH_3$ | $COOC_2H_4OCH_3$ | |
| 65 | $CF_3$ | $NO_2$ | $NO_2$ | 4 Cl | $CH_3$ | $CONHC_2H_4OCH_3$ | |
| 66 | Cl | CN | Cl | 4 Cl | $CH_3$ | $COOCH_3$ | Smp. 113–4° |
| 67 | Cl | CN | Cl | 4 Cl | $CH_3$ | $COOC_2H_5$ | |
| 68 | Cl | CN | Cl | 4 Cl | $CH_3$ | COOH | Smp. 115° |
| 69 | Cl | CN | Cl | 4 Cl | $CH_3$ | $COOisoC_4H_9$ | Oel |
| 70 | Cl | CN | Cl | 4 Cl | $CH_3$ | $COOnC_4H_9$ | Oel |
| 71 | Cl | CN | Cl | 4 Cl | H | $COOCH_3$ | Smp. 114–116° |

| No. | C | D | B | Ep | $R_1$ | A | physikalische Konstante |
|---|---|---|---|---|---|---|---|
| 72 | Cl | CN | Cl | 4 Cl | $CH_3$ | $CH_3$<br>\|<br>$COOCH_2CHC_2H_3$ | Oel |
| 73 | Cl | CN | Cl | 4 Br | $CH_3$ | $COOnC_3H_7$ | Oel |
| 74 | Cl | CN | Cl | 4 Br | $CH_3$ | $CH_3$<br>\|<br>$COOCH_2CHC_2H_5$ | Oel |
| 75 | Cl | CN | Br | 4 Br | $CH_3$ | $COOCH_3$ | Smp. 107–9° |
| 76 | Cl | CN | – | 5 Cl | $CH_3$ | $COOCH_3$ | $n_D^{25}$.5736 |
| 77 | Cl | $NO_2$ | – | 4 Cl | $CH_3$ | $COOCH_3$ | |
| 78 | Cl | $NO_2$ | – | 5 Cl | $CH_3$ | $COOCH_3$ | |
| 79 | Cl | $NO_2$ | Cl | 4 Cl | $CH_3$ | $COOCH_3$ | Harz |
| 80 | Cl | Cl | CN | 4 Cl | $CH_3$ | $COOCH_3$ | |
| 81 | Cl | Cl | I | 4 Cl | $CH_3$ | $COOCH_3$ | |
| 82 | Cl | Cl | Cl | 4 Cl | $CH_3$ | $COOCH_3$ | Kp. 190°/0.001 Torr |
| 83 | Cl | Cl | Cl | 4 Cl | $CH_3$ | COO 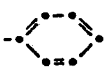 | |
| 84 | Cl | Cl | Cl | 4 Cl | $CH_3$ | $COOCH_2CH{=}CH_2$ | Oel |
| 85 | Cl | Cl | $NO_2$ | 4 Cl | $CH_3$ | $COOC_2H_4Cl$ | |
| 86 | Cl | Cl | $NO_2$ | 4 Cl | $CH_3$ | $COOCH_2$ 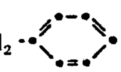 | |
| 87 | Cl | Cl | $NO_2$ | Cl | $CH_3$ | $COOisoC_3H_7$ | |
| 88 | Cl | Cl | – | 4 Cl | $CH_3$ | $COOCH_3$ | Oel |
| 89 | Cl | Cl | $NO_2$ | 4 Cl | $CH_3$ | $COOCH_3$ | Smp. 95–6° |
| 90 | Cl | Cl | Cl | 4 Br | $CH_3$ | $COOCH_3$ | Kp. 200°/0.001 Torr |
| 91 | Cl | Br | $NO_2$ | 2 Cl 4 Cl | H | $CONH_2$ | |
| 92 | Cl | Br | $NO_2$ | 2 Cl 4 Cl | $CH_3$ | $COOCH_2CH{=}C(CH_3)_2$ | |
| 93 | Cl | Br | $NO_2$ | 2 Cl 4 Cl | H | COOH | |
| 94 | Cl | Br | $NO_2$ 2 | Cl 4 Cl | H | COS 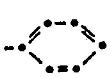 | |

| No. | C | D | B | Ep | $R_1$ | A | physikalische Konstante |
|---|---|---|---|---|---|---|---|
| 95 | Cl | Br | $NO_2$ 2 | Cl 4 Cl | H | $COSC_2H_4SCH_3$ | |
| 96 | Cl | Br | $NO_2$ 2 | Cl 4 Cl | H | $COOC_2H_4SCH_3$ | |
| 97 | Cl | Br | $NO_2$ 2 | Cl 4 Cl | H | CN | |
| 98 | Br | Br | $NO_2$ | 4 Cl | $CH_3$ | $COOCH_3$ | |
| 99 | Br | Br | $NO_2$ | 4 Cl | $CH_3$ | $CONHCH_3$ | |
| 100 | Br | Br | $NO_2$ | 4 Cl | $CH_3$ | 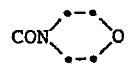 | |
| 101 | Cl | Cl | Cl | 4 Cl | $CH_3$ | $COOCH_2-CH=CH_2$ | Oel Kp 0,001 200° |
| 102 | Cl | Cl | Cl | 4 Cl | $CH_3$ | $CON(CH_3)OCH_3$ | Oel |
| 103 | Cl | Cl | Cl | 4 Cl | $CH_3$ | $COC_2H_4Cl$ | Oel |
| 104 | Cl | Cl | Cl | 4 Cl | $CH_3$ | $COOCHC_2H_5$<br>$\vert$<br>$CH_3$ | Oel Kp 0,001 210° |
| 105 | Cl | Cl | Cl | 4 Cl | $CH_3$ | $COOC_2H_4OCH_3$ | Oel Kp 0,001 230° |
| 106 | Cl | Cl | Cl | 4 Cl | $CH_3$ | $COOC_2H_4CN$ | Oel |
| 107 | Cl | Cl | Cl | 4 Cl | $CH_3$ | $CONHC_2H_4OCH_3$ | Oel |
| 108 | Cl | Cl | Cl | 4 Cl | $CH_3$ | $COOC_2H_4N(CH_3)_2$ | Oel |
| 109 | $NO_2$ | – | – | 4 Cl | $CH_3$ | $COOCH_3$ | Oel |
| 110 | $NO_2$ | – | $NO_2$ | 4 Cl | $CH_3$ | $COOCH_3$ | |
| 111 | $NO_2$ | – | – | 5 Cl | $CH_3$ | $COOCH_3$ | |
| 112 | $NO_2$ | Cl | Cl | 4 Cl | $CH_3$ | $COOCH_3$ | Smp. 98–99° |
| 113 | $NO_2$ | Cl | – | 5 Cl | $CH_3$ | $COOCH_3$ | Harz |
| 114 | CN | – | $NO_2$ | 4 Cl | $CH_3$ | $COOCH_3$ | |

Die Herstellung erfindungsgemässer Mittel erfolgt in an sich bekannter Weise durch inniges Vermischen und Vermahlen von Wirkstoffen der allgemeinen Formel I mit geeigneten Trägerstoffen und/oder Verteilungsmitteln, gegebenenfalls unter Zusatz von gegenüber den Wirkstoffen inerten Antischaum-, Netz-, Dispersions- und/oder Lösungsmitteln. Die Wirkstoffe können in den folgenden Aufarbeitungsformen vorliegen und angewendet werden:

Feste Aufarbeitungsformen:
Stäubemittel, Streumittel, Granulate, Umhüllungsgranulate, Imprägniergranulate und Homogengranulate;

In Wasser dispergierbare Wirkstoffkonzentrate:
Spritzpulver (wettable powder), Pasten, Emulsionen; Emulsionskonzentrate

Flüssige Aufarbeitungsformen:
Lösungen

Die Wirkstoffkonzentrationen betragen in den erfindungsgemässen Mitteln 1 bis 80 Gewichtsprozent und können gegebenenfalls bei der Anwendung auch in geringen Konzentrationen wie etwa 0,05 bis 1% vorliegen.

Den beschriebenen erfindungsgemässen Mitteln lassen sich andere biozide Wirkstoffe oder Mittel beimischen. So können die neuen Mittel ausser den genannten Verbindungen der allgemeinen Formel I z.B. Insektizide, Fungizide, Bakterizide, Fungistatika, Bakteriostatika, Nematozide oder weitere Herbizide zur Verbreiterung des Wirkungspektrums enthalten.

Die Herstellung erfindungsgemässer Mittel wird durch die folgenden Formulierungsbeispiele näher erläutert.

Spritzpulver

Zur Herstellung eines a) 70%igen und b) 10%igen Spritzpulvers werden folgende Bestandteile verwendet:

a) 70 Teile eines der Wirkstoffe der Formel I,
5 Teile Natriumdibutylnaphtylsulfonat,
3 Teile Naphthalinsulfonsäuren-Phenolsulfonsäuren-Formaldehyd-Kondensat 3:2:1,
10 Teile Kaolin,
12 Teile Champagne-Kreide;

b) 10 Teile eines der Wirkstoffe der Formel I,
3 Teile Gemisch der Natriumsalze von gesättigten Fettalkoholsulfaten,
5 Teile Naphthalinsulfonsäuren-Formaldehyd-Kondensat,
82 Teile Kaolin.

Der angegebene Wirkstoff wird auf die entsprechenden Trägerstoffe (Kaolin und Kreide) aufgezogen und anschliessend vermischt und vermahlen mit den übrigen Bestandteilen. Man erhält Spritzpulver von vorzüglicher Benetzbarkeit und Schwebefähigkeit. Aus solchen Spritzpulvern können durch Verdünnen mit Wasser Suspensionen von 0,1—8% Wirkstoff erhalten werden, die sich zur Unkrautbekämpfung in Pflanzenkulturen eignen.

Paste

Zur Herstellung einer 45%igen Paste werden folgende Stoffe verwendet:

45 Teile eines Wirkstoffes der Formel I,

5 Teile Natriumaluminiumsilikat,

14 Teile Cetylpolyglykoläther mit 8 Mol Aethylenoxid,

1 Teil Oleylpolyglykoläther mit 5 Mol Aethylenoxid,

2 Teile Spindeloel,

10 Teile Polyäthylenglykol,

23 Teile Wasser.

Der Wirkstoff wird mit den Zuschlagstoffen in dazu geeigneten Geräten innig vermischt und vermahlen. Man erhält eine Paste, aus der sich durch Verdünnen mit Wasser Suspensionen jeder gewünschten Konzentration herstellen lassen.

Emulsionskonzentrat

Zur Herstellung eines 25%iges Emulsionskonzentrates werden

25 Teile eines Wirkstoffes der Formel I,

10 Teile einer Mischung von Nonylphenolpolyoxyäthylen und Calciumdodecylbenzolsulfonat,

10 Teile Cyclohexanon

55 Teile Xylol

miteinander vermischt. Dieses Konzentrat kann mit Wasser zu Emulsionen auf geeignete Konzentrationen von z.B. 0,1% verdünnt werden. Solche Emulsionen eignen sich zur Bekämpfung von Unkräutern in Kulturpflanzungen.

Die neuen Phenoxy-$\alpha$-phenoxyalkancarbonsäuren und ihre Derivate der Formel I, sowie die sie enthaltenden Mittel besitzen eine ausgezeichnete selektiv-herbizide Wirkung gegen Unkräuter in den verschiedensten Kulturpflanzenbeständen. Sie haben ebenfalls eine das Pflanzenwachstum regulierende Wirkung.

Obwohl die neuen Wirkstoffe der Formel I bei pre- und post-emergenter Anwendung wirksam sind, scheint die post-emergente Applikation als Kontaktherbizide den Vorzug zu verdienen, obwohl auch der pre-emergente Einsatz von Interesse ist. Bevorzugt werden die neuen Wirkstoffe als z.B. 25%ige Spritzpulver oder z.B. 20%-ige emulgierbare Konzentrate formuliert und mit Wasser verdünnt, auf die Pflanzenbestände post-emergent appliziert.

Zum Nachweis der Brauchbarkeit als Herbizide (pre- und post-emergent) und zum Beweis der Ueberlegenheit gegenüber bekannten Wirkstoffen ähnlicher Struktur dienen folgende Testmethoden:

Pre-emergente Herbizid-Wirkung (Keimhemmung)

Im Gewächshaus wird unmittelbar nach der Einsaat der Versuchspflanzen in Saatchalen die Erdoberfläche mit einer wässerigen Dispersion der Wirkstoffe, erhalten aus einem 25%-igen Emulsionkonzentrat resp. aus einem 25%-igen Spritzpulver mit Wirkstoffen, die wegen ungenügender Löslichkeit nicht als Emulsionskonzentrat hergestellt werden können, behandelt. Es wurden vier verschiedene Konzentrationsreihen angewendet, entsprechend 4, 2, 1 und 0,5 kg Wirksubstanz pro Hektar. Die Saatschalen werden im Gewächshaus bei 22—25°C und 50—70% rel. Luftfeuchtigkeit gehalten und der Versuch nach 3 Wochen ausgewertet und die Resultate nach folgender Notenskala bonitiert:

1 = Pflanzen nicht gekeimt oder total abgestorben

2—3 = sehr starke Wirkung

4—6 = mittlere Wirkung

7—8 = geringe Wirkung

9 = keine Wirkung (wie unbehandelte Kontrolle)

als Versuchspflanzen dienen beispielweise:

| | |
|---|---|
| hordeum (Gerste) | setaria italica |
| triticum (Weizen) | echinochloa crus galli |
| zea (Mais) | beta vulgaris |
| sorghum hybr. (Hirse) | sida spinosa |
| oryza (Reis) | sesbania exaltata |
| glycine (Soja) | amaranthus retroflexus |
| gossypium (Baumwolle) | sinapis alba |
| avena fatua | ipomoea purpurea |
| lolium perenne | galium aparine |
| alopecurus myosuroides | pastinaca sativa |
| bromus tectorum | rumex sp. |
| cyperus esculentus | chrysanthemum leucum. |
| rottboellia exaltata | abutilon sp. |
| digitaria sanguinalis | solanum nigrum |

Post-emergente Herbizid-Wirkung (Kontaktherbizid)

Eine grössere Anzahl (mindestens 7) Unkräuter und Kulturpflanzen, sowohl monocotyle wie dicotyle, wurden nach dem Auflaufen (im 4-bis-6-Blattstatium) mit einer wässrigen Wirkstoffdispersion in Dosierungen von 0,06; 0,125; 0,25; 0,5 kg Wirksubstanz pro Hektar auf die Pflanzen gespritzt und diese bei 24°—26°C und 45—60% rel. Luftfeuchtigkeit gehalten. Mindestens 15 Tage nach Behandlung wird der Versuch ausgewertet und das Ergebnis wie im pre-emergent-Versuch nach derselben Notenskala bonitiert.

Die geprüften Verbindungen gemäss vorliegender Erfindung zeigten auf einigen Pflanzen ausgeprägte kontaktherbizide Wirkung und auf vielen Pflanzen Wachstumsstillstand als Symptom der wachstumshemmenden Eigenschaften.

Wuchshemmung bei Gräsern

In Kunststoffschalen mit Erde-Torf-Sand-Gemisch (6:3:1) wurden Samen der Gräser Lolium perenne, Poa pratensis, Festuca ovina und Dactylis glomerata ausgesät und normal bewässert. Die aufgelaufenen Gräser wurden wöchentlich bis auf 4 cm Höhe zurückgeschnitten und 40 Tage nach der Aussaat und 1 Tag nach dem letzten Schnitt mit wässerigen Spritzbrühen eines Wirkstoffs der Formel I bespritzt. Die Wirkstoffmenge betrug umgerechnet 5 kg Aktivsubstanz pro Hektar. 10 und 21 Tage nach Applikation wurde das Wachstum der Gräser beurteilt.

Wuchshemmung bei Getreide

In Kunststoffbechern wurde Sommerweizen (Triticum aestivum), Sommergerste (Hordeum vulgare) und Roggen (Secale) in sterilisierter Erde angesät und im Gewächshaus gezogen. Die Getreidesprösslinge werden 5 Tage nach Aussaat mit einer Spritzbrühe des Wirkstoffs behandelt. Die Blattapplikation entsprach 6 kg Wirkstoff pro Hektar. Die Auswertung erfolgt nach 21 Tage.

Die erfindungsgemässen Wirkstoffe bewirken eine merkliche Wuchshemmung sowohl bei den Gräsern wie beim Getreide.

**Patentansprüche**

1. Phenoxy-phenoxy-alkancarbonsäurederivate der Formel I

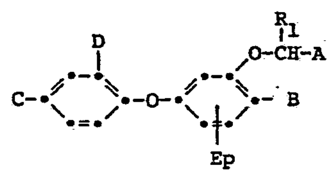 (I)

In dieser Formel bedeuten

$R_1$ Wasserstoff, $C_1$—$C_4$ Alkyl, $C_2$—$C_3$ Alkoxyalkyl

A die Cyanogruppe oder einen Rest —COR,

R ein Rest —$OR_3$, $SR_4$, —$NR_5R_6$ oder —$N{=}R_2$

$R_3$ Wasserstoff oder das Kation einer Base $\frac{1}{m} M^{m\oplus}$

M ein Alkali-, Erdalkali-Kation oder ein Fe, Cu-, Zn-, Mn, Ni-Kation oder einen Ammonio-Rest

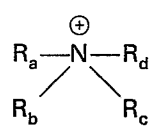

m als ganze Zahl 1, 2 oder 3 die Wertigkeit des Kations berücksichtigt, während $R_a$, $R_b$, $R_c$ und $R_d$ unabhängig voneinander Wasserstoff, Benzyl oder einen gegebenenfalls durch —OH, —$NH_2$ oder $C_1$—$C_4$ Alkoxy substituierten $C_1$—$C_4$ Alkylrest bedeuten,

weiter bedeuten

$R_3$ einen $C_1$—$C_{12}$ Alkyl-Rest, der unsubstituiert oder gegebenenfalls substituiert ist durch Halogen, Nitro, Cyano, $C_1$—$C_8$ Alkoxy, $C_1$—$C_8$ Alkylthio, $C_2$—$C_8$ Alkanoyl, Bis ($C_1$—$C_4$ alkyl) amino, Tris ($C_1$—$C_4$ alkyl)ammonio, $C_3$—$C_8$ Cycloalkyl, $C_3$—$C_8$ Cycloalkenyl, gegebenenfalls auch durch einen unsubstituierten oder seinerseits durch Halogen, $C_1$—$C_4$-Alkyl, $C_1$—$C_4$-Alkoxy ein- oder mehrfach substituierten Phenyl-, Phenoxy-oder 5—6 gliedrigen heterocyclischen Rest mit 1 bis 3 Heteroatomen;

— einen unsubst. oder subst. $C_3$—$C_{18}$ Alkenylrest;

— einen $C_3$—$C_8$ Alkinyl-Rest;

— einen gegebenenfalls durch Halogen oder $C_1$—$C_4$-Alkyl substituierten $C_3$—$C_{12}$ Cycloalkyl-Rest;

— einen Phenylrest, der unsubstituiert oder durch Halogen, $C_1$—$C_4$-Alkyl, $C_1$—$C_4$-Alkoxy, $C_1$—$C_4$-Alkylthio, $NO_2$, $CF_3$, ein- oder mehrfach substituiert ist;

— einen 5- bis 6-gliedrigen heterocyclischen Ring mit 1 bis 3 Heteroatomen,

$R_4$ dasselbe wie $R_3$,

$R_5$ und $R_6$ unabhängig voneinander je Wasserstoff oder einen *Alkylrest* der gegebenenfalls durch Hydroxyl oder Alkoxy substituiert sein kann, *Alkoxy, Alkenyl, Alkinyl, Phenyl* oder *Benzyl* wobei die Phenylringe ein- oder mehrfach durch Halogen, Alkyl, Alkoxy, Alkylthio, Cyano oder Nitro substituiert sein können oder

$R_5$ und $R_6$ zusammen mit dem Stickstoffatom, an das sie gebunden sind, einen 5—6 gliedrigen Heterocyclus, der auch noch ein zweites Heteroatom, Stickstoff, Sauerstoff oder Schwefel enthalten kann,

$R_2$ einen durch eine Doppelbindung an das Stickstoffatom gebunden, vorzugsweise verzweigte Alkylenrest,

B Wasserstoff oder Halogenatom oder ein Cyano- oder Nitrorest,

C und D je Wasserstoff, Halogen oder einen Cyano-, Nitro- oder Trifluormethylrest,

E ein Halogenatom oder ein $C_1$—$C_4$ Alkylrest und

P eine Zahl 1 oder 2 bedeuten.

2. Die Verbindungen der Formel I, Anspruch 1, in denen C die Trifluormethylgruppe und D Wasserstoff bedeutet, während A, B, E, p und $R_1$ die unter Formel I gegebene Bedeutung haben.

3. Die Verbindungen der Formel I, Anspruch 1, in denen C die Trifluormethylgruppe und D ein Halogenatom bedeutet, während A, B, C, p und $R_1$ die unter Formel I gegebene Bedeutung haben.

4. Die Verbindungen der Formel I, Anspruch 1, in denen C und D Halogenatome bedeuten, während A, B, E, p und $R_1$ die unter Formel I gegebene Bedeutung haben.

5. Die Verbindungen der Formel I, Anspruch 1, in denen C die Trifluormethylgruppe und D eine Cyano oder Nitrogruppe bedeutet während A, B, E, p und $R_1$ die unter Formel I gegebene Bedeutung haben.

6. Die Verbindungen der Formel I, Anspruch 1, in denen C ein Halogenatom und D die Cyanogruppe bedeuten, während A, B, E, p und $R_1$ die unter Formel I gegebene Bedeutung haben.

7. Die Verbindungen der Formel I, Anspruch 1, in denen B, C, D und E je ein Halogenatom und p die Zahl 1 bedeuten, während A die unter Formel I gegebene Bedeutung hat.

8. Die Verbindungen der Formel I, Anspruch 1, in denen B, D und E je ein Halogenatom bedeuten, C die Trifluormethylgruppe, p die Zahl 1 und A die unter Formel I, Anspruch 1 gegebene Bedeutung hat.

9. Als Verbindung gemäss Anspruch 1 $\alpha$-[3-(2',4'-Dichlorphenoxy)-4,6-dichlorphenoxy)-propionsäure-methylester.

10. Als Verbindung gemäss Anspruch 1, $\alpha$-[3-(2'-Chlor-4-trifluormethylphenoxy)-4,6-dichlorphenoxy]-propionsäure-methylester.

11. Als Verbindung gemäss Anspruch 1, $\alpha$-[3-(2'-Chlor-4'-trifluormethylphenoxy)-4,6-dichlorphenoxy]-propionsäure-isopropylester.

12. Als Verbindung gemäss Anspruch 1, $\alpha$-[3-(2'-Chlor-4'-trifluormethylphenoxy)-4,6-dichlorphenoxy]-propionsäure-N-methyl-N-methoxyamid.

13. Als Verbindung gemäss Anspruch 1, $\alpha$-[3-(2'-Chlor-4'-trifluormethylphenoxy)-4,6-dichlorphenoxy]-propionsäure-N-($\beta$-methoxyäthyl)-amid.

14. Als Verbindung gemäss Anspruch 1, $\alpha$-[3-(2'-Chlor-4'-trifluormethylphenoxy)-4-chlor-6-cyanophenoxy]-propionsäure-methylester.

15. Verfahren zur Herstellung von Phenoxy-phenoxyalkancarbonsäurederivaten der Formel I, Anspruch 1, dadurch gekennzeichnet, dass man ein entsprechend substituiertes Halogenbenzol der Formel II

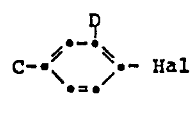

(II)

worin Hal ein Halogenatom, vorzugsweise Chlor oder Brom bedeutet und C und D die unter Formel I, Anspruch 1 gegebene Bedeutung haben, mit einem entsprechend substituierten Hydroxyphenoxyalkancarbonsäure-derivat der Formel III

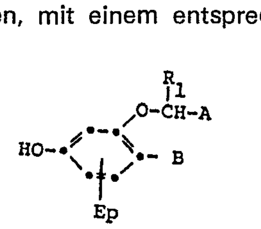

(III)

worin

A, E, p und $R_1$ die unter Formel I, Anspruch 1 gegebene Bedeutung haben in an sich bekannter

Weise, in einem Lösungsmittel in Gegenwart einer Base umsetzt.

16. Verfahren zur Herstellung von Phenoxy-phenoxy-alkancarbonsäurederivaten der Formel I, Anspruch 1, dadurch gekennzeichnet, dass man einen entsprechend substituierten 3-Hydroxy-diphenyläther der Formel IV

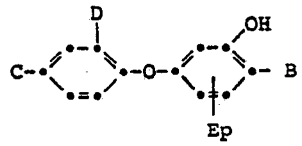

(IV)

worin A, C, D, E und p die unter Formel I, Anspruch 1 gegebene Bedeutung haben, mit einem $\alpha$-Halogencarbonsäurederivat der Formel V

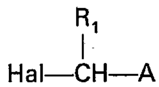

(V)

worin Hal ein Halogenatom, vorzugsweise Chlor oder Brom darstellt und A und $R_1$ die unter Formel I, Anspruch 1 gegebene Bedeutung haben, in an sich bekannter Weise in einem Lösungsmittel, in Gegenwart einer Base umsetzt.

17. Verfahren zur Herstellung von Phenoxy-phenoxy-alkancarbonsäurederivaten der Formel I, Anspruch 1, dadurch gekennzeichnet, dass man eine Verbindung der Formel I, in der A die freie Carboxylgruppe bedeutet, durch Behandeln mit einer anorganischen oder organischen Base in ein Salz überführt.

18. Verfahren zur Herstellung von Phenoxy-phenoxy-alkancarbonsäurederivaten der Formel I, Anspruch 1, dadurch gekennzeichnet, dass man eine Verbindung der Formel I in der A die freie Carboxylgruppe bedeutet, in ein Säurehalogenid verwandelt und dieses anschliessend durch Reaktion mit einem Alkanol, einem Thiol oder einem Amid, in ein anderes Säurederivat entsprechend der Definition von A umsetzt.

19. Herbizides und das Pflanzenwachstum regulierendes Mittel, dadurch gekennzeichnet, dass es als Wirkstoff mindestens ein Phenoxyphenoxy-alkancarbonsäurederivat der Formel I, Anspruch 1 enthält.

20. Die Verwendung der Phenoxy-phenoxy-alkancarbonsäurederivate der Formel I, Anspruch 1, oder sie enthaltenden Mittel als Herbizide.

21. Die Verwendung der Phenoxy-phenoxy-alkancarbonsäurederivate der Formel I, Anspruch 1, oder sie enthaltenden Mittel zur Regulierung des Pflanzenwachstums.

## Claims

1. A phenoxy-phenoxy-alkanecarboxylic acid derivative of the formula I

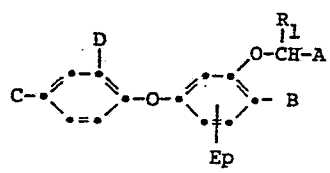

(I)

in which

$R_1$ is hydrogen, $C_1$—$C_4$ alkyl or $C_2$—$C_3$ alkoxyalkyl,
A is the cyano group or a radical —COR, wherein
R is a radical —$OR_3$, $SR_4$—$NR_5R_6$ or —$N{=}R_2$, wherein

$R_3$ is hydrogen or the cation of a base $\frac{l}{m}M^{m\oplus}$ wherein

M is an alkali metal cation or alkaline-earth metal cation, or a Fe, Cu, Zn, Mn or Ni cation, or an ammonium group

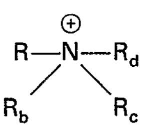 and ,

m as integer 1, 2 or 3 takes into account the valency of the cation, whilst $R_a$, $R_b$, $R_c$ and $R_d$

independently of one another are hydrogen, benzyl or a $C_1$—$C_4$ alkyl group unsubstituted or substituted by —OH, —$NH_2$ or $C_1$—$C_4$ alkoxy, and in addition

$R_3$ is a $C_1$—$C_{12}$ alkyl which is unsubstituted or substituted by halogen, nitro, cyano, $C_1$—$C_8$ alkoxy $C_1$—$C_8$ alkylthio, $C_2$—$C_8$ alkanoyl, bis ($C_1$—$C_4$ alkyl) amino, tris ($C_1$—$C_4$ alkyl) ammonium, $C_3$—$C_8$ cycloalkyl, $C_3$—$C_8$ cycloalkenyl, or by a phenyl, phenoxy or 5- or 6-membered heterocyclic radical having 1 to 3 hetero atoms, each of which is unsubstituted or for its part mono- or polysubstituted by halogen, $C_1$—$C_4$ alkyl or $C_1$—$C_4$ alkoxy, or $R_3$ is an unsubstituted or substituted $C_3$—$C_{18}$ alkenyl group, a $C_3$—$C_8$ alkynyl group, a $C_3$—$C_{12}$ cycloalkyl group unsubstituted or substituted by halogen or $C_1$—$C_4$ alkyl, or $R_3$ is a phenyl group which is unsubstituted or mono- or polysubstituted by halogen, $C_1$—$C_4$ alkyl, $C_1$—$C_4$ alkoxy, $C_1$—$C_4$ alkylthio, $NO_2$ or $CF_3$, or $R_3$ is a 5- or 6-membered heterocyclic ring having 1 to 3 hetero atoms, and

$R_4$ is the same as $R_3$,

$R_5$ and $R_6$ independently of one another are each hydrogen, or an alkyl group which can be unsubstituted or substituted by hydroxyl or alkoxy, or they are each alkoxy, alkenyl, alkynyl, phenyl or benzyl, and the phenyl rings can be mono- or polysubstituted by halogen, alkyl alkoxy, alkylthio, cyano or nitro, or

$R_5$ and $R_6$ together with the nitrogen atom to which they are bound can form a 5—6-membered heterocycle, which can also contain a second hetero atom, nitrogen, oxygen or sulfur,

$R_2$ is a preferably branched-chain alkylene group bound by a double bond to the nitrogen atom,

B is hydrogen or a halogen atom, or a cyano or nitro group,

C and D are each hydrogen, halogen, or a cyano, nitro or trifluoromethyl group,

E is a halogen atom or a $C_1$—$C_4$ alkyl group, and

p is the number 1 or 2.

2. A compound of the formula I, Claim 1, in which C is the trifluoromethyl group, and D is hydrogen, and A, B, E, p and $R_1$ are as defined under the formula I.

3. A compound of the formula I, Claim 1, in which C is the trifluoromethyl group, D is a halogen atom, and A, B, C, p and $R_1$ are as defined under the formula I.

4. A compound of the formula I, Claim 1, in which C and D are halogen atoms, and A, B, E, p and $R_1$ are as defined under the formula I.

5. A compound of the formula I, Claim 1, in which C is the trifluoromethyl group, D is a cyano or nitro group, and A, B, E, p and $R_1$ are as defined under the formula I.

6. A compound of the formula I, Claim 1, in which C is a halogen atom, D is the cyano group, and A, B, E, p and $R_1$ are as defined under the formula I.

7. A compound of the formula I, Claim 1, in which B, C, D and E are each a halogen atom, p is the number 1, and A is as defined under the formula I.

8. A compound of the formula I, Claim 1, in which B, D and E are each a halogen atom, C is the trifluoromethyl group, p is the number 1, and A is as defined under the formula I, Claim 1.

9. As a compound according to Claim 1: $\alpha$-[3-(2',4'-dichlorophenoxy)-4,6-dichlorophenoxy)-propionic acid methyl ester.

10. As a compound according to Claim 1: $\alpha$-[3-(2'-chloro-4-trifluoromethylphenoxy)-4,6-dichlorophenoxy]-propionic acid methyl ester.

11. As a compound according to Claim 1: $\alpha$-[3-(2'-chloro-4'-trifluoromethylphenoxy)-4,6-dichlorophenoxy]-propionic acid isopropyl ester.

12. As a compound according to Claim 1: $\alpha$-[3-(2'-chloro-4'-trifluoromethylphenoxy)-4,6-dichlorophenoxy]-propionic acid-N-methyl-N-methoxyamide.

13. As a compound according to Claim 1: $\alpha$-[3-(2'-chloro-4'-trifluoromethylphenoxy)-4,6-dichlorophenoxy]-propionic acid-N-($\beta$-methoxyethyl)-amide.

14. As a compound according to Claim 1: $\alpha$-[3-(2'-chloro-4'-trifluoromethylphenoxy)-4-chloro-6-cyanophenoxy]-propionic acid methyl ester.

15. A process for producing phenoxy-phenoxy-alkane-carboxylic acid derivatives of the formula I, Claim 1, which process comprises reacting in a manner known per se an appropriately substituted halogenobenzene of the formula II

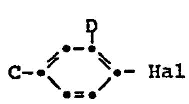

(II)

in which "Hal" is a halogen atom, preferably chlorine or bromine, and C and D are as defined under the formula I, Claim 1, with an appropriately substituted hydroxyphenoxyalkanecarboxylic acid derivative of the formula III

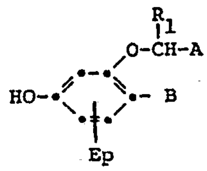

(III)

in which A, E, p and $R_1$ are as defined under the formula I, Claim 1, in a solvent in the presence of a base.

16. A process for producing phenoxy-phenoxy-alkane-carboxylic acid derivatives of the formula I, Claim 1, which process comprises reacting in a manner known per se an appropriately substituted 3-hydroxy-diphenyl ether of the formula IV

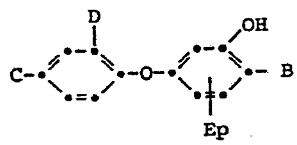

(IV)

in which A, C, D, E and p are as defined under the formula I, Claim 1, with an $\alpha$-halogenocarboxylic acid derivative of the formula V

$$\mathrm{Hal{-}\overset{\displaystyle R_1 \atop |}{C}H{-}A} \qquad (V)$$

in which "Hal" is a halogen atom, preferably chlorine or bromine, and A and $R_1$ are as defined under the formula I, Claim 1, in a solvent in the presence of a base.

17. A process for producing phenoxy-phenoxy-alkane-carboxylic acid derivatives of the formula I, Claim 1, which process comprises converting a compound of the formula I in which A is the free carboxyl group, by treatment with an inorganic or organic base, into a salt.

18. A process for producing phenoxy-phenoxy-alkane-carboxylic acid derivatives of the formula I, Claim 1, which process comprises converting a compound of the formula I, in which A is the free carboxyl group, into an acid halide, and subsequently converting this, by reaction with an alkanol, a thiol or an amide, into another acid derivative corresponding to the definition of A.

19. A herbicide and composition regulating plant growth, which contains as active substance at least one phenoxy-phenoxy-alkanecarboxylic acid derivative of the formula I, Claim 1.

20. The use of the phenoxy-phenoxy-alkanecarboxylic acid derivatives of the formula I, Claim 1, or of compositions containing them, as herbicides.

21. The use of the phenoxy-phenoxy-alkanecarboxylic acid derivatives of the formula I, Claim 1, or of compositions containing them, for regulating plant growth.

**Revendications**

1. Dérivés d'acides phénoxy-phénoxy-alcanoïques de formule I

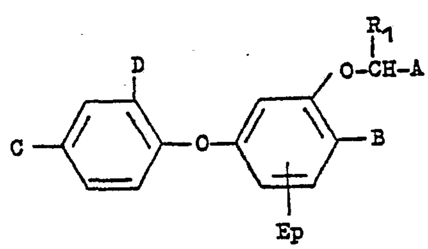

(I)

dans cette formule

$R_1$ représente l'hydrogène, un groupe alkyle en $C_1$—$C_4$, alcoxyalkyle en $C_2$—$C_3$,
A représente le groupe cyano ou un radical —COR,
R représente un radical —$OR_3$, $SR_4$ —$NR_5R_6$ ou —N=$R_2$,

$R_3$ représente l'hydrogène ou le cation d'une base $\frac{1}{m} M^{m\oplus}$

M représente un cation alcalin, alcalino-terreux ou un cation de Fe, de Cu, de Zn, de Mn, de Ni ou un radical ammonio

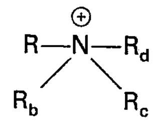

m est un nombre entier égal à 1, 2 ou 3 qui correspond à la valence du cation, cependant que $R_a$, $R_b$, $R_c$ et $R_d$ représentent chacun, indépendamment les uns des autres, l'hydrogène, un groupe benzyle ou un radical alkyle en $C_1$—$C_4$ portant éventuellement des substituants OH, $NH_2$ ou alcoxy en $C_1$—$C_4$, en outre

$R_3$ représente un reste alkyle en $C_1$—$C_{12}$ non substitué ou éventuellement substitué par des halogènes, des groupes nitro, cyano, alcoxy en $C_1$—$C_8$, alkylthio en $C_1$—$C_8$, alkanoyle en $C_2$—$C_8$, bis-(alkyle en $C_1$—$C_4$)-amino, tris-(alkyle en $C_1$—$C_4$)-ammonio, cycloalkyle en $C_3$—$C_8$, cycloalcényle en $C_3$—$C_8$, le cas échéant également par un radical phényle, phénoxy ou un radical hétérocyclique à 5—6 chaînons et 1 à 3 hétéroatomes, non substitué ou lui-même mono- ou poly-substitué par des halogènes, des groupes alkyles en $C_1$—$C_4$, alcoxy en $C_1$—$C_4$;

— un radical alcényle en $C_3$—$C_{18}$ non substitué ou substitué;

— un radical alcynyle en $C_3$—$C_8$,

— un radical cycloalkyle en $C_3$—$C_{12}$ éventuellement substitué par des halogènes ou des groupes alkyles en $C_1$—$C_4$;

— un radical phényle non substitué ou mono- ou polysubstitué par des halogènes, des groupes alkyles en $C_1$—$C_4$, alcoxy en $C_1$—$C_4$, alkylthio en $C_1$—$C_4$, $NO_2$, $CH_3$,

— un noyau hétérocyclique de 5 à 6 chaînones contenant 1 à 3 hétéroatomes,

$R_4$ a la même signification que $R_3$

$R_5$ et $R_6$ représentent chacun, indépendamment l'un de l'autre, l'hydrogène ou un radical *alkyle* qui peut éventuellement être substitué par des groupes hydroxy ou alcoxy, *alcoxy, alcényle, alcynyle, phényle* ou *benzyle,* les noyaux phényles pouvant être mono- ou poly-substitués par des halogènes, des groupes alkyle, alcoxy, alkylthio, cyano ou nitro, ou bien $R_5$ et $R_6$ forment ensemble et avec l'atome d'azote auquel ils sont reliés un hétérocycle à 5—6 chaînons qui peut également contenir un deuxième hétéroatome, d'azote, d'oxygène ou de soufre,

$R_2$ représente un radical alkylène de préférence ramifié fixé par une double liaison à l'atome d'azote,

B représente l'hydrogène ou un atome d'halogène ou un radical cyano ou nitro,

C et D représentent chacun l'hydrogène, un halogène, un radical cyano, nitro ou trifluorométhyle,

E représente un atome d'halogène ou un radical alkyle en $C_1$—$C_4$ et

p est égal à 1 ou 2.

2. Les composés de formule I, revendication 1, dans lesquels C représente le groupe trifluorométhyle et D l'hydrogène, A, B, R, p et $R_1$ ayant les significations indiquées en référence à la formule I.

3. Les composés de formule I, revendication 1, dans lesquels C représente le groupe trifluorométhyle et D un atome d'halogène, A, B, C, p et $R_1$ ayant les significations indiquées en référence à la formule I.

4. Les composés de formule I, revendication 1, dans lesquels C et D représentent des atomes d'halogènes, A, B, E, p et $R_1$ ayant les significations indiquées en référence à la formule I.

5. Les composés de formule I, revendication 1, dans lesquels C représente le groupe trifluorométhyle et D un groupe cyano ou nitro, A, B, E, p et $R_1$ ayant les significations indiquées en référence à la formule I.

6. Les composés de formule I, revendication 1, dans lesquels C représente un atome d'halogène et D le groupe cyano, A, B, E, p et $R_1$ ayant les significations indiquées en référence à la formule I.

7. Les composés de formule I, revendications 1, dans lesquels B, C, D et E représentent chacun un atome d'halogène et p est égal à 1, A ayant les significations indiquées en référence à la formule I.

8. Les composés de formule I, revendication 1, dans lesquels B, D et E représentent chacun un atome d'halogène, C le groupe trifluorométhyle, p est égal à 1 et A a les significations indiquées en référence à la formule I, revendication 1.

9. En tant que composé selon la revendication 1, 1'α-[3-(2',4'-dichlorophénoxy)-4,6-dichlorophénoxy]-propionate de méthyle.

10. En tant que composé selon la revendication 1, 1'α-[3-(2'-chloro-4-trifluorométhylphénoxy)-4,6-dichlorophénoxy]-propionate de méthyle.

11. En tant que composé selon la revendication 1, 1'α-[3-(2'-chloro-4'-trifluorométhylphénoxy)-4,6-dichlorophénoxy]-propionate d'isopropyle.

12. En tant que composé selon la revendication 1, l'$\alpha$-[3-(2'-chloro-4'-trifluorométhylphénoxy)-4,6-dichlorophénoxy]-propio-N-méthyl-N-méthoxyamide.

13. En tant que composé selon la revendication 1, l'$\alpha$-[3-(2'-chloro-4'-trifluorométhylphénoxy)-4,6-dichlorophénoxy]-propio-N-($\beta$-méthoxyéthyl)-amide.

14. En tant que composé selon la revendication 1, l'$\alpha$-[3-(2'-chloro-4'-trifluorométhylphénoxy)-4-chloro-6-cyanophénoxy]-propionate de méthyle.

15. Procédé de préparation des dérivés d'acides phénoxy-phénoxy-alcanoïques de formule I, revendication 1, caractérisé en ce que l'on fait réagir en présence d'une base, dans un solvant, de manière connue en soi, un halogénobenzène, portant les substituants correspondants et répondant à la formule II

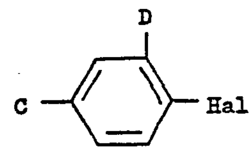

(II)

dans laquelle Hal représente un atome d'halogène, de préférence de chlore ou de brome, et C et D ont les significations indiquées en référence à la formule I, revendication 1, avec un dérivé d'acide hydroxyphénoxy-alcanoïque portant les substituants correspondants et répondant à la formule III

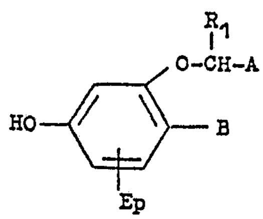

(III)

dans laquelle

A, E, p et $R_1$ ont les significations indiquées en référence à la formule I, revendication 1.

16. Procédé de préparation des dérivés d'acides phénoxy-phénoxy-alcanoïques de formule I, revendication 1, caractérisé en ce que l'on fait réagir en présence d'une base, dans un solvant, de manière connue en soi, un éther 3-hydroxy-diphénylique portant les substituants correspondants et répondant à la formule IV

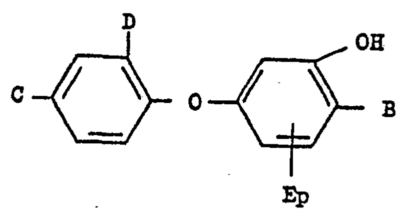

(IV)

dans laquelle A, C, D, E et p ont les significations indiquées en référence à la formule I, revendication 1, avec un dérivé d'acide $\alpha$-halogénocarboxylique de formule V

$$\text{Hal}-\overset{\displaystyle R_1}{\overset{|}{C}H}-A \qquad \text{(V)}$$

dans laquelle Hal représente un atome d'halogène, de préférence de chlore ou de brome, et A et $R_1$ ont les significations indiquées en référence à la formule I, revendication 1.

17. Procédé de préparation des dérivés d'acides phénoxy-phénoxy-alcanoïques de formule I, revendication 1, caractérisé en ce que l'on convertit un composé de formule I dans laquelle A représente le groupe carboxyle libre en un sel par traitement à l'aide d'une base minérale ou organique.

18. Procédé de préparation de dérivés d'acides phénoxy-phénoxy-alcanoïques de formule I, revendication 1, caractérisé en ce que l'on convertit un composé de formule I dans laquelle A représente le groupe carboxyle libre en un halogénure d'acide qu'on convertit ensuite en un autre dérivé d'acide correspondant à la définition de A par réaction avec un alcanol, un thiol ou un amide.

19. Produit herbicide et régulateur de la croissance des végétaux caractérisé en ce qu'il contient, en tant que substance active, au moins un dérivé d'acide phénoxy-phénoxy-alcanoïque de formule I, revendication 1.

20. L'utilisation des dérivés d'acide phénoxy-phénoxy-alcanoïques de formule I, revendication 1, ou de produits en contenant, en tant qu'herbicides.

21. L'utilisation des dérivés d'acides phénoxy-phénoxy-alcanoïques de formule I, revendication 1, ou de produits en contenant, pour la régulation de la croissance des végétaux.